Europäisches Patentamt

European Patent Office

Office européen des brevets

(11) Publication number: **0 421 697 A2**

## EUROPEAN PATENT APPLICATION

(21) Application number: 90310702.7

(22) Date of filing: 28.09.90

(51) Int. Cl.⁵: **A61K 49/00**

(30) Priority: 29.09.89 US 414791

(43) Date of publication of application:
10.04.91 Bulletin 91/15

(84) Designated Contracting States:
AT BE CH DE DK ES FR GB GR IT LI LU NL SE

(71) Applicant: SMITHKLINE BEECHAM
CORPORATION
P.O. Box 7929 1 Franklin Plaza
Philadelphia Pennsylvania 19101(US)

(72) Inventor: Kinter, Lewis B.
200 East Eagle Road
Havertown, Pennsylvania 19083(US)
Inventor: Lenkinski, Robert E.
828 Lindale Avenue
Drexel Hill, Pennsylvania 19026(US)
Inventor: Rycyna, Robert E.
649 S. Henderson Road
King of Prussia, Pennsylvania 19406(US)
Inventor: Sarkar, Susanta K.
56 Constitution Court
Wayne, Pennsylvania 19087(US)

(74) Representative: Giddings, Peter John, Dr. et al
SmithKline Beecham, Corporate Patents,
Mundells
Welwyn Garden City, Hertfordshire AL7
1EY(GB)

(54) Contrast agents and methods for enhancing magnetic resonance imaging.

㊳ Chelates of certain paramagnetic metals, particularly ytterbium diethylenetriamine pentaacetic acid (Yb-DTPA), have been found to alter or enhance contrast of magnetic resonance (MR) images by causing susceptibility-induced spin dephasing in the tissue to be imaged, but causing substantially no dipole-dipole interactions. These contrast agents are particularly useful in monitoring fluid flow using fast imaging sequences, such as gradient echo imaging, particularly glomerular filtration rate (renal clearance) to detect renal pathologies, such as glomerulonephritis, and cerebral blood flow to detect focal blood-brain barrier defects, which could allow early diagnosis of stroke.

EP 0 421 697 A2

Xerox Copy Centre

# CONTRAST AGENTS AND METHODS FOR ENHANCED MAGNETIC RESONANCE IMAGING

## Field of the Invention

The present invention is directed to methods and materials for enhancing the contrast of magnetic resonance images (MRI). More particularly, the invention is directed to new techniques for disease diagnosis using contrast agents in MRI.

## Background of the Invention

Magnetic Resonance Imaging (MRI) is now clinically used to diagnose diseased states and their response to therapy. In general, the contrast in MR images is generated due to the differences in relaxation behavior of the protons in water in the tissue or organ. However, as with other imaging modalities, specificity or sensitivity in MRI can also be improved by use of appropriate contrast agents, in this case magnetopharmaceuticals.

As a result, there is a need to evaluate contrast agents for clinical use in MRI. One class of compounds generally used for this purpose is paramagnetic metal complexes. See German Patent No. 31 29 906 of Schering AG (Gries et al.) and U.S. Patent No. 4,687,659 of Salutar, Inc. (Quay). These agents work almost exclusively by changing the relaxation behavior of water protons through dipole-dipole interactions between the spin of unpaired electrons of the paramagnetic metal ion and nuclear spin of protons. One such agent commonly used in clinic is gadolinium-diethylenetriamine pentaacetic acid (Gd-DTPA) complex.

It has recently been reported that the contrast generated in the MR image with the administration of Gd-DTPA is not only due to the dipole-dipole interaction but also arises from susceptibility-induced spin dephasing. See Villringer, A. et al., "Dynamic Imaging With Lanthanide Chelates in Normal Brain: Contrast Due to Magnetic Susceptibility Effects,", Magnetic Resonance in Medicine , 6:164-174 (1988). However, it would be desirable to have contrast agents which work exclusively with one or the other mechanism.

## Summary of the Invention

According to the present invention, contrast agents are provided for use in magnetic resonance (MR) imaging which are substantially non-toxic (physiologically tolerable) and cause susceptibility-induced spin dephasing in tissue being imaged, and cause substantially no dipole-dipole interactions with water protons of the tissue. Preferred contrast agents used in the methods of the invention are chelates of ytterbium (Yb), particularly ytterbium-diethylenetriamine pentaacetic acid (Yb-DTPA) or physiologically acceptable salts thereof. The contrast agents are particularly useful for monitoring bodily fluid flows, such as glomerular filtration rate (GFR) to detect renal pathologies and cerebral blood flow to detect focal blood-brain barrier defects, using fast MR imaging sequences, such as gradient echo imaging.

## Brief Description of the Drawings

For the purpose of illustrating the invention, there is shown in the drawing an illustration of a form which is presently preferred; it being understood, however, that this invention is not limited to the precise embodiment illustrated.

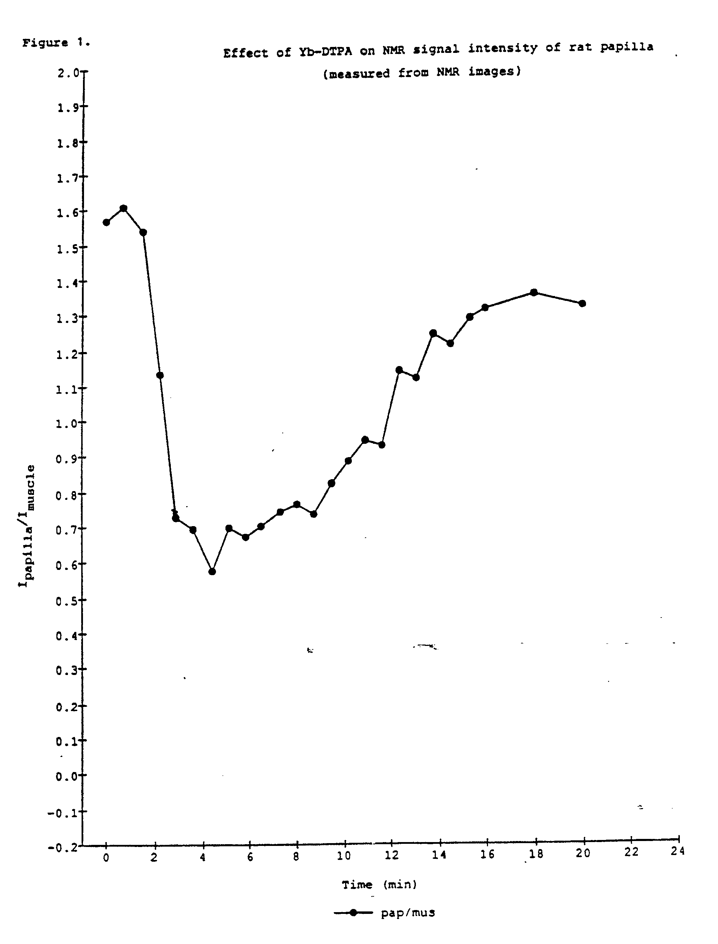

Figure 1 is a graph showing the contrast produced by Yb-DTPA, in the form of MR signal intensity change, in MR imaging of a normal rat kidney over time.

## Detailed Description of the Preferred Embodiments

Diethylenetriamine pentaacetic acid (DTPA) and similar chelates of paramagnetic metals, particularly gadolinium, are well known in the art as contrast agents for MRI. Methods for making them are known in the literature, including, for example, German Patent No. 31 29 906. However, there are some drawbacks for the use of Gd-DTPA in some cases, as it causes huge drop in signal intensity because of the effects of both the dipole-dipole and susceptibility-induced mechanism. This results in some odd effects, e.g., overestimating the size of some lesions in the tissue.

According to the present invention, it was found that certain paramagnetic metals provide MRI contrast enhancement almost exclusively by causing susceptibility-induced spin dephasing. The preferred paramagnetic metal for use in the present invention is ytterbium (Yb).

The contrast agents of the present invention and methods of using them will be described hereafter with particular reference to Yb-DTPA, it being understood that other chelates of paramagnetic metals having susceptibility-induced spin dephasing effects similar to Yb-DTPA without causing substantial dipole-dipole interactions can be used in the following methods in the same manner.

Any of a wide variety of physiologically acceptable complexing agents may be used to form chelates of the paramagnetic metals for use in the present invention. In addition to DTPA, these complexing agents include, for example, diamide DTPA, DTPA dimeglumine, etc. Methods of making chelates of gadolinium are well known in the art, and ytterbium or other paramagnetic metals may be chelated in essentially the same manner. See Allen, R.C. (ed.), Annual Reports in Medicinal Chemistry , Vol. 24, Chapter 28, pp. 265-276, Academic Press, Inc. (1989) for a good review of current contrast media.

The chelate is first dissolved in a physiologically tolerable solvent for in vivo use. Suitable solvents include, for example, water, serum, albumin solutions, and saline, but other solvents will be evident to those skilled in the art. The solvent should make the chelate suitable for intravenous (IV) or interperitoneal (IP) injection or infusion, although oral administration may ultimately be feasible.

The chelate should be present in the solution in an amount to provide about 20 to 200, and preferably about 50, mmol of the paramagnetic metal per liter of solution. The solution is then introduced into the animal tissue, as indicated above, at a dose of about 0.05 to 0.2 mmol per kg body weight. A preferred dose is about 0.17 mmol chelate per kg body weight. Much higher doses will provide too dark an image and may be toxic, while much lower doses may not provide sufficient contrast. An IV infusion rate of about 5-10 ml/min is satisfactory.

After administration of the chelate solution to the subject, imaging with any conventional MR imaging system may be begun. Preferably, a fast imaging sequence, such as gradient echo imaging is used. In such techniques, images are collected approximately every 20 seconds or so, so that the MR system may provide a graph over time which shows the clearance of the contrast agent through the tissue being imaged. This graph plots the ratio of signal intensity of region of interest to signal intensity of surrounding muscle tissue over time, as shown, for example in Figure 1, discussed below.

The classical method for measuring renal clearance, for example, is an atomic measurement of inulin in tissue samples. In order to demonstrate that renal elimination of Yb-DTPA is also a good measure of glomerular filtration rate (GRF), the following experiments were conducted:

### Preparation of Yb-DTPA

The disodium salt of Yb-DTPA was prepared using the following procedure. A solution containing 3.94 g (0.01 mol) diethylenetriamine pentaacetic acid, 1.97 g (0.005 mol) $Yb_2O_3$ and 250 ml $H_2O$ were heated to reflux overnight. Then, 0.8 g (0.02 mol) NaOH was added and heated at reflux for an additional 24 hours. The cooled solution was filtered to remove undissolved material, and the filtrate was concentrated to an oil using a rotary evaporator. The oil was triturated with ethanol and the resulting solid washed with ether. Following repeated recrystallizations from water and ethanol (1:10, v/v), the solid was dried under reduced pressure. Elemental analysis reported stoichiometric proportions of $Yb^{+3}$ and DTPA in the solid.

### Comparative Example

Male Sprague-Dawley rats (200 to 350 g; Charles River Breeding Laboratories, Raleigh, NC) were anesthetized with Inactin (100 mg/kg, i.p.; Byk Gulden). A jugular vein and femoral artery were isolated and cannulated with PE-50 polyethylene tubing (Clay-Adams). The femoral artery cannula was connected to a pressure transducer (Gould-Statham Model P23id) for blood pressure and heart rate recording. The jugular vein cannula was connected to an infusion pump (Sage Model 352) and an infusion of inulin (Inutest, Laevasan-Gesellschaft; 10% in 0.9% NaCl) and ytterbium-diethyltriaminepentaacetic acid (Yb-DTPA in water 6.0 mg/ml) was initiated at a rate of μl/min. A femoral vein was isolated and cannulated with PE-50 tubing for collection of blood samples. A small midline incision was made and the apex of the urinary bladder exposed and cannulated (PE-50 tubing) for collection of urine. The rat was then allowed to equilibrate for 1 hour prior to the start of the study protocol.

Following equilibration, three 20-min urine samples were collected; 1.0 ml blood samples were collected in heparinized tubes at the midpoint of the first and third collection periods ($C_1$ and $C_3$). At the end of the experiment, the rats were killed by pentobarbital overdose.

Urine volumes were measured gravimetrically (specific gravity = 1) and 50 μl was removed for determination of inulin and Yb-DTPA concentrations. Inulin concentrations in plasma and urine samples were determined according to the method of Heyrosky (1956). Yb concentrations were determined by inductively-coupled plasma emission spectrometry using a Model JY-38P (Instruments SA, Inc.) and a grating of 3600 lines/mm. The 369.42 nm Yb line and 5 one-second measurements were taken at the end of each sample aspiration to provide an average reading (Bumbier, et al., 1984). For determination of Yb concentrations, 20 $\mu$l of either plasma or urine were diluted into 3 ml of sterile water. Plasma concentrations of inulin and Yb-DTPA for $C_2$ were estimated from values measured for $C_1$ and $C_3$.

From these procedures, the renal clearances of inulin and Yb were calculated, normalized for differences in body weight (ml/100 g BW) and compared using linear regression analysis (RS/1, Bolt, Beranek and Newman Software Products Corp.).

Plasma inulin concentrations ranged from 540 to 790 $\mu$g/ml; inulin concentrations in urine ranged from 18.4 to 117 mg/ml. Plasma Yb concentrations ranged from 0.057 to 0.091 ppm; Yb concentrations in urine ranged from 2.1 to 16.2 ppm. The renal clearances of inulin and Yb ranged from 750 to 1154 ml $(100 \text{ g BW-min})^{-1}$ and from 829 to 1364 ml $(100 \text{ g BW-min})^{-1}$, respectively. Mean ($\pm$ SEM) $C_{in}$ and $C_{Yb}$ were $956 \pm 37$ and $1101 \pm 47$ ml $(100 \text{ g BW-min})^{-1}$, respectively ($p<0.05$). Correlation of simultaneous $C_{in}$ and $C_{Yb}$ values was highly significant ($p = 0.000004$). The data were determined to best fit the equation $y = 1.145247x + 5.830056$. Thus, the renal clearance of Yb-DTPA parallels the renal clearance of inulin over the plasma concentration range tested, and appears to slightly overestimate (~15%) the inulin clearance. The basis for the small but consistent differences in $C_{in}$ and $C_{Yb}$ is not elucidated by this study. The results could be interpreted to show a small but statistically significant secretory component in the renal handling of Yb-DTPA. No suggestion of saturation of this mechanism is apparent over the plasma concentration range tested, suggesting that $Tm_{Yb\text{-}DTPA}$ was not exceeded. On the other hand, the difference in $C_{in}$ and $C_{Yb}$ may result from differences in analytical methodologies.

We conclude that the principal route (~85%) of renal elimination of Yb-DTPA is via glomerular filtration. Thus, the renal clearance of Yb-DTPA, like the renal clearance of inulin, is a useful indicator of glomerular function.

## MR Imaging Example

As in the above comparative example, rats were provided with an infusion line and strapped in an MRI magnet GE SIGNA 1.5T. 1 ml of a 50 mM solution of Yb-DTPA in saline was injected (intravenously) as a bolus. Images were collected every 20 seconds and the signal intensity from the rat papilla ($I_{papilla}$) and signal intensity for the surrounding rat muscle ($I_{muscle}$) were measured. The ratios of these intensities ($I_{papilla}/I_{muscle}$) were then plotted against time as shown in Figure 1. The sharp decrease in the ratio and the slow rise shows the injection of the Yb-DTPA and its clearance by glomerular filtration in the normal rat kidney, since the intensity of the muscle does not change. The area under the curve is an index of GFR.

In the case of kidney blockage due to glomerulonephritis or other renal pathologies, there is a blockage of the papilla, which would be shown by flattening out of the curve of Figure 1, i.e., there would be little change in the $I_{papilla}/I_{muscle}$ ratio, indicating that renal clearance was impaired.

The susceptibility induced mechanism for MRI contrast agents is a new mode of contrast agent action in MR imaging. It therefor may potentially provide information which differs from that obtained from the currently used contrast agents. One possible use of this type of agent will be in monitoring flow, e.g., identification of focal blood-brain barrier defects may be facilitated. Monitoring of cerebral blood volume and flow would allow detection of stroke at an early stage. Use of this agent to diagnose glomerulonephritis and other important renal pathologies, such as nephrotoxicity, renal hypertrophy, and renal ischemia or infarction, appears feasible from the above examples and would also be important for diabetics, who often have GFR problems.

The present invention also appears to be promising in connection with other MRI procedures using fast imaging sequences and could provide clear advantages in generating contrast for these. In general, the measurement or monitoring of perfusion (e.g., blood supply or vascularization of tumors) appears to be enhanced with the contrast agents of the present invention.

The present invention may be embodied in other specific forms without departing from the spirit or essential attributes thereof and, accordingly, reference should be made to the appended claims, rather than to the foregoing specification, as indicating the scope of the invention.

## Claims

1. A method for increasing the contrast of a magnetic resonance (MR) image of kidney tissue in vivo to detect renal pathologies by monitoring glomerular filtration rate, comprising the steps of providing a physiologically tolerable solution of a chelate of ytterbium (Yb), introducing said solution into a fluid in the kidney tissue to be imaged, allowing said Yb chelate to interact with said tissue to cause susceptibility-induced spin dephasing, and imaging the tissue with an MR system to obtain a contrast enhanced MR image.

2. A method according to claim 1 wherein said chelate is ytterbium-diethylenetriamine pentaacetic acid (Yb-DTPA) or a physiologically acceptable salt thereof.

3. A method according to claim 1 wherein said Yb chelate alters the contrast of the image of the kidney papilla with respect to the surrounding kidney muscle.

4. A method according to claim 1 wherein said MR system employs a fast imaging sequence.

5. A method according to claim 4 wherein said fast imaging sequence is gradient echo imaging.

6. A method of diagnosing renal pathologies using magnetic resonance (MR) imaging comprising providing a physiologically tolerable solution of a chelate of a paramagnetic metal which causes susceptibility-induced spin dephasing in said kidney, allowing said paramagnetic metal to cause susceptibility induced spin dephasing in said kidney, and imaging the kidney papilla to monitor the glomerular filtration rate therefrom using the altered signal intensity of the papilla caused by said paramagnetic metal.

7. A method according to claim 6 wherein said paramagnetic metal is ytterbium.

8. A method according to claim 6 wherein said chelate is ytterbium diethylenetriamine pentaacetic acid (Yb-DTPA) or a physiologically acceptable salt thereof.

9. A method according to claim 6 wherein said MR imaging uses a fast imaging sequence.

10. A method according to claim 9 wherein said fast imaging sequence is gradient echo imaging.

11. A method according to claim 6 wherein said chelate is introduced at a dose of about 0.05 to about 0.2 mmol/kg body weight.

12. A method according to claim 6 wherein said chelate causes substantially no dipole-dipole interaction between the paramagnetic metal and water protons of the kidney tissue.

Figure 1.

Effect of Yb-DTPA on NMR signal intensity of rat papilla
(measured from NMR images)